# EUROPEAN PATENT APPLICATION

(11) **EP 4 620 582 A1**
(43) Date of publication of application: **24.09.2025**
(21) Application number: 25161662.9
(22) Date of filing: 04.03.2025
(51) Int. Cl.: B06B 1/06

(54) **ULTRASOUND PROBE**

(30) Priority: 18.03.2024 JP 2024041926
(71) Applicant: FUJIFILM Corporation, Tokyo 106-8620 (JP)
(72) Inventor: HASE, Takatoshi, Kanagawa, 258-8538 (JP)
(74) Representative: Klunker IP Patentanwälte PartG mbB

(57) **Abstract**

Provided is an ultrasound probe in which even in a case where the number of channels of a piezoelectric element is increased, reliability is not impaired and it is not necessary to change acoustic design.

An ultrasound probe includes: a flexible circuit board disposed between the backing material and the plurality of piezoelectric elements, in which each of the plurality of piezoelectric elements consisting of a laminate in which a signal electrode layer, a piezoelectric body portion, and a ground electrode layer are sequentially laminated in a lamination direction on a surface of the flexible circuit board, the signal electrode layer is electrically connected to the flexible circuit board, the flexible circuit board is longer than the piezoelectric body portion in an elevation direction, first members consisting of an adhesive in which abrasive grains are dispersed are disposed at end parts of the laminate in the elevation direction, and the ground electrode layer is electrically connected to the flexible circuit board via metal foil that extends from a surface of the laminate to surfaces of the first members and side surfaces of the first members in the elevation direction.

## Description

### BACKGROUND OF THE INVENTION

### 1. Field of the Invention

The present invention relates to an ultrasound probe in which a plurality of piezoelectric elements are arranged in an array along an azimuth direction.

### 2. Description of the Related Art

Hitherto, in the medical field, an ultrasound diagnostic apparatus using ultrasound images has been put into practical use. In general, in this type of ultrasound diagnostic apparatus, an ultrasound image is generated by transmitting an ultrasound beam from an ultrasound probe toward a subject, receiving an ultrasound echo from the subject through the ultrasound probe, and electrically processing a reception signal thereof.

The ultrasound probe usually has a plurality of piezoelectric elements arranged in an array along a so-called azimuth direction on a backing material. JP2001-276060A and JP2001-298795A disclose an ultrasound probe in which a flexible circuit board is disposed between a backing material and a plurality of piezoelectric elements in order to extract a signal from the plurality of piezoelectric elements.

Signal electrode layers of the plurality of piezoelectric elements facing the flexible circuit board are connected to the flexible circuit board, whereby the signals are extracted from the plurality of piezoelectric elements. In addition, metal foil is connected to a ground electrode layer of the plurality of piezoelectric elements disposed on the side opposite to the signal electrode layer, and the plurality of piezoelectric elements are grounded using the metal foil.

### SUMMARY OF THE INVENTION

However, in recent years, due to the increase in the number of channels of the piezoelectric element caused by the decrease in the pitch and the increase in the field of view of the piezoelectric element due to the increase in the frequency, a flexible circuit board long in an elevation direction is required to extract a signal, and the length of the flexible circuit board in the elevation direction tends to be longer than the length of the piezoelectric element.

In this manner, in a case where the length of the flexible circuit board in the elevation direction is longer than the length of the piezoelectric element, a gap is likely to be generated between the metal foil connected to the ground electrode layer of the piezoelectric element and end parts of the piezoelectric element in the elevation direction, and in a case of dividing the piezoelectric element into a plurality of piezoelectric elements by dicing using the cutting blade, the cutting blade is likely to flutter, so that the piezoelectric element may be damaged, whereby the reliability of the ultrasound probe may be impaired.

In a case where the piezoelectric element is made longer in the elevation direction in accordance with the flexible circuit board, the bandwidth shape is changed, and it is necessary to change the acoustic design.

In addition, in a case where the number of channels of the piezoelectric element is increased, the cutting blade used for dicing is likely to be abraded, and the cut cross section of the piezoelectric element is damaged, which may impair the reliability of the ultrasound probe.

The present invention has been made in order to solve such a problem in the related art, and an object of the present invention is to provide an ultrasound probe in which even in a case where the number of channels of a piezoelectric element is increased, reliability is not impaired and it is not necessary to change acoustic design.

According to the following configuration, the above object can be achieved.
[1] An ultrasound probe in which a plurality of piezoelectric elements are arranged in an array on a backing material along an azimuth direction, the ultrasound probe comprising:
   a flexible circuit board disposed between the backing material and the plurality of piezoelectric elements,
   in which each of the plurality of piezoelectric elements consisting of a laminate in which a signal electrode layer, a piezoelectric body portion, and a ground electrode layer are sequentially laminated in a lamination direction on a surface of the flexible circuit board,
   the signal electrode layer is electrically connected to the flexible circuit board,
   the flexible circuit board is longer than the piezoelectric body portion in an elevation direction,
   first members consisting of an adhesive in which abrasive grains are dispersed are disposed at end parts of the laminate in the elevation direction, and
   the ground electrode layer is electrically connected to the flexible circuit board via metal foil that extends from a surface of the laminate to surfaces of the first members and side surfaces of the first members in the elevation direction.
[2] The ultrasound probe according to [1], in which the first members are each disposed at both end parts of the laminate in the elevation direction.
[3] The ultrasound probe according to [1] or [2], in which the first members have an acoustic attenuation rate equal to or higher than an acoustic attenuation rate of the backing material.
[4] The ultrasound probe according to any one of [1] to [3], in which the first members consist of a vitreous adhesive or a ceramic adhesive in which any of a white alumina abrasive, a green silicon carbide, or a resinoid is dispersed as the abrasive grains.
[5] The ultrasound probe according to any one of [1] to [4], in which second members having an acoustic attenuation rate higher than an acoustic attenuation rate of the first members are disposed between the end parts of the laminate in the elevation direction and the first members.
[6] The ultrasound probe according to [5], in which the second members consist of a buffer material.
[7] The ultrasound probe according to [6], in which the buffer material consists of a silicone resin or an epoxy resin.
[8] The ultrasound probe according to any one of [1] to [7], further comprising:
   a dematching layer disposed between the flexible circuit board and the plurality of piezoelectric elements and having an acoustic impedance higher than an acoustic impedance of the piezoelectric body portionwherein a dematching layer having an acoustic impedance higher than an acoustic impedance of the piezoelectric body portion is disposed between the flexible circuit board and the plurality of piezoelectric elements.

According to the invention, a flexible circuit board is disposed between the backing material and the plurality of piezoelectric elements, each of the plurality of piezoelectric elements consists of a laminate in which a signal electrode layer, a piezoelectric body portion, and a ground electrode layer are sequentially laminated in a lamination direction on a surface of the flexible circuit board, the signal electrode layer is electrically connected to the flexible circuit board, the flexible circuit board is longer than the piezoelectric body portion in an elevation direction, first members consisting of an adhesive in which abrasive grains are dispersed are disposed at end parts of the laminate in the elevation direction, and the ground electrode layer is electrically connected to the flexible circuit board via metal foil that extends from a surface of the laminate to surfaces of the first members and side surfaces of the first members in the elevation direction. Therefore, even in a case where the number of channels of the piezoelectric element is increased, the ultrasound probe that does not impair reliability and does not need to change acoustic design is realized.

### BRIEF DESCRIPTION OF THE DRAWINGS

Fig. 1 is a perspective view showing a part of an ultrasound probe according to Embodiment 1 of the present invention.
Fig. 2 is a cross-sectional view of the ultrasound probe according to Embodiment 1 of the present invention.
Fig. 3 is a cross-sectional view of the ultrasound probe according to Embodiment 2 of the present invention.
Fig. 4 is a cross-sectional view of the ultrasound probe according to Embodiment 3 of the present invention.
Fig. 5 is a cross-sectional view of the ultrasound probe according to Embodiment 4 of the present invention.

### DESCRIPTION OF THE PREFERRED EMBODIMENTS

Hereinafter, embodiments of the present invention will be described with reference to the accompanying drawings.

The description of configuration requirements described below is given on the basis of a representative embodiment of the present invention, but the present invention is not limited to such an embodiment.

Note that, in the present specification, a numerical range represented by using "to" means a range including numerical values before and after "to" as a lower limit value and an upper limit value.

In the present specification, "same" and "identical" include an error range generally allowed in the technical field.

### Embodiment 1

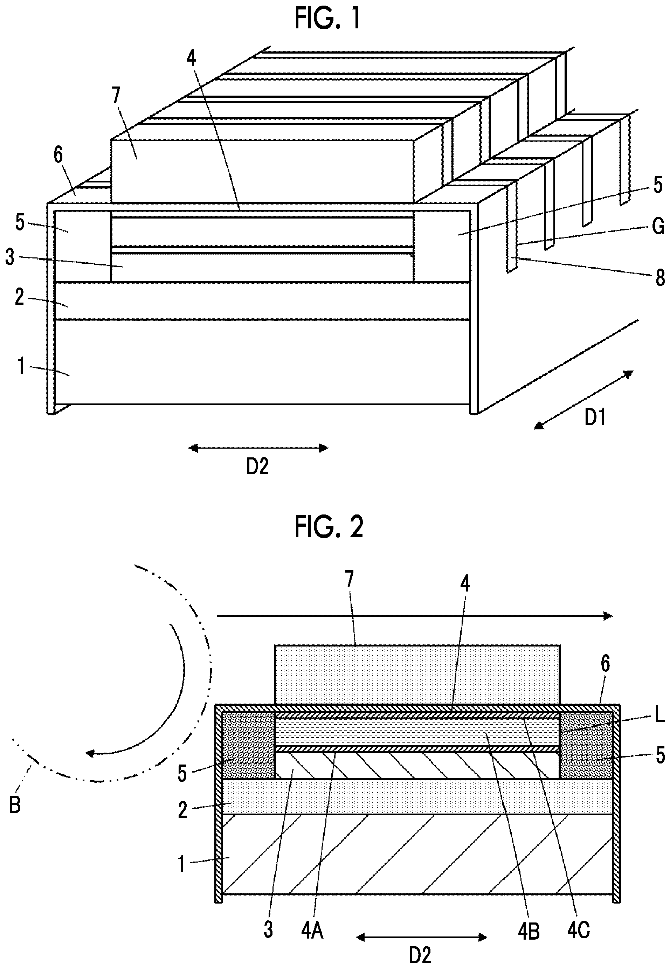

Fig. 1 shows a configuration of an ultrasound probe according to Embodiment 1 of the present invention. The ultrasound probe includes a backing material 1, a flexible circuit board 2 disposed on the backing material 1, a dematching layer 3 disposed on the flexible circuit board 2, and a plurality of piezoelectric elements 4 disposed on the dematching layer 3.

The flexible circuit board 2 is formed to be longer than the dematching layer 3 and the piezoelectric element 4 in the elevation direction D2, and a pair of first members 5 formed on the flexible circuit board 2 are disposed at both end parts of the dematching layer 3 and the piezoelectric element 4 in the elevation direction D2.

The ultrasound probe further includes metal foil 6 disposed on the plurality of piezoelectric elements 4 and the pair of first members 5, and an acoustic matching layer 7 disposed on the metal foil 6. The metal foil 6 is bent rearward toward the backing material 1 at the corners of the pair of first members 5 and extends to the side surface of the backing material 1 along the side surfaces of each of the first members 5 in the elevation direction D2.

A direction from the backing material 1 toward the acoustic matching layer 7 is referred to as "frontward", and a direction from the acoustic matching layer 7 toward the backing material 1 is referred to as "rearward".

The plurality of piezoelectric elements 4 are arranged in an array along the azimuth direction D1, and a groove G is formed between each of the plurality of piezoelectric elements 4. The groove G is formed to reach the surface of the flexible circuit board 2 from the acoustic matching layer 7, and thus, the dematching layer 3, the pair of first members 5, and the acoustic matching layer 7 are also divided into a plurality of pieces along the azimuth direction D1, similarly to the plurality of piezoelectric elements 4. Further, the metal foil 6 of the portions disposed on the upper part of the plurality of piezoelectric elements 4 and the side surfaces of the pair of first members 5 is also divided into a plurality of pieces along the azimuth direction D1 in accordance with the plurality of piezoelectric elements 4.

The inside of the groove G is filled with the filler 8.

The backing material 1 absorbs the ultrasonic waves emitted from the plurality of piezoelectric elements 4 rearward, and is formed of a material in which a filler including ferrite, tungsten, manganese, or the like is dispersed in a base material including a rubber material, an epoxy resin, a polyurethane resin, or the like.

The flexible circuit board 2 is a circuit board for leading out electrodes of the plurality of piezoelectric elements 4, and has a multilayer structure in which a plurality of wiring layers are formed.

The dematching layer 3 is a layer for reflecting the ultrasonic waves, which are emitted from the plurality of piezoelectric elements 4 rearward to the front side, and is formed of a material having an acoustic impedance higher than an acoustic impedance of a piezoelectric body portion described later, which constitutes the piezoelectric element 4, for example, tungsten, tungsten carbide, tungsten oxide, tantalum, or the like.

The acoustic matching layer 7 is a layer for matching an acoustic impedance of the plurality of piezoelectric elements 4 and the subject to facilitate incidence of the ultrasonic waves into the subject, and can be formed of a material having an acoustic impedance that is lower than the acoustic impedance of the piezoelectric body portion of the piezoelectric element 4 and is higher than an acoustic impedance of the subject. In addition, the acoustic matching layer 7 can also have a multi-layer structure in which the acoustic impedance is gradually decreased from the piezoelectric element 4 toward the subject.

The filler 8 is a filler for fixing the positions and postures of the dematching layer 3, the piezoelectric element 4, and the acoustic matching layer 7 adjacent to each other in the azimuth direction D1, and is formed of, for example, a silicone resin, an epoxy resin, or the like.

As shown in Fig. 2, each piezoelectric element 4 is formed of a laminate L in which a signal electrode layer 4A, a piezoelectric body portion 4B, and a ground electrode layer 4C are sequentially laminated on the surface of the dematching layer 3 frontward (lamination direction).

The piezoelectric body portion 4B is formed of a known piezoelectric material, expands and contracts by the application of a voltage to release an ultrasonic wave, and expands and contracts by the propagation of a so-called ultrasound echo from the outside to generate an electric signal. Examples of the piezoelectric material include piezoelectric ceramics such as lead zirconate titanate (PZT), and a polymer material such as polyvinylidene fluoride (PVDF).

The signal electrode layer 4A is used for applying a voltage to the piezoelectric body portion 4B and extracting an electric signal generated by the expansion and contraction of the piezoelectric body portion 4B due to the propagation of the ultrasound echo. The signal electrode layer 4A is electrically connected to a wiring layer (not shown) of the flexible circuit board 2 via the dematching layer 3 having conductivity. Specifically, the dematching layer 3 has electrode layers (not shown) on a surface facing the plurality of piezoelectric elements 4 and a surface facing the flexible circuit board 2, respectively, and the signal electrode layer 4A of each piezoelectric element 4 is electrically connected to a wiring layer (not shown) of the flexible circuit board 2 by using the conductivity of the electrode layers disposed on both surfaces of the dematching layer 3 and the dematching layer 3.

The ground electrode layer 4C is used for setting a reference potential of the piezoelectric body portion 4B to a predetermined ground potential, and is in contact with the metal foil 6 disposed on the upper part of the piezoelectric element 4 to be electrically connected thereto.

Each of the pair of first members 5 is formed of an adhesive in which abrasive grains are dispersed. Specifically, the first members 5 can be formed from a vitreous adhesive or a ceramic adhesive in which any of a white alumina abrasive, a green silicon carbide, or a resinoid is dispersed as the abrasive grains.

In addition, since the first members 5 are in contact with the side surface of the piezoelectric element 4 in the elevation direction D2, it is desirable that the first members 5 have an acoustic attenuation rate equal to or higher than an acoustic attenuation rate of the backing material 1 for the purpose of suppressing the influence on the acoustic characteristics of the piezoelectric element 4.

The abrasive grains included in the first members 5 are used for performing a polishing treatment on a cutting blade B used in a case of dividing the piezoelectric element 4 into the plurality of piezoelectric elements 4.

The pair of first members 5 have a height corresponding to the sum of the thicknesses of the dematching layer 3 and the piezoelectric element 4 from the surface of the flexible circuit board 2 toward the front side, and the surfaces of the first members 5 facing the front side form the same surface as the surface of the ground electrode layer 4C of the piezoelectric element 4. Therefore, the metal foil 6 extends in a planar shape from the piezoelectric element 4 to the first members 5, is bent rearward at the corners of the first members 5, and extends rearward along the side surfaces of the first members 5 in the elevation direction D2.

Since the ultrasound probe according to Embodiment 1 of the present invention includes the pair of first members 5, even in a case where the flexible circuit board 2 is formed to be longer than the dematching layer 3 and the piezoelectric element 4 in the elevation direction D2, the ultrasound probe is configured such that a gap is not generated at both end parts of the dematching layer 3 and the piezoelectric element 4 in the elevation direction D2.

Therefore, it is possible to prevent the piezoelectric element 4 from being damaged due to fluttering of the cutting blade B in a case of dividing the piezoelectric element 4 into the plurality of piezoelectric elements 4 by dicing using the cutting blade B. In addition, it is not necessary to increase the lengths of the dematching layer 3 and the piezoelectric element 4 in the elevation direction D2 in accordance with the length of the flexible circuit board 2, and it is not necessary to change the acoustic design.

Further, since the first members 5 have a height corresponding to the sum of the thicknesses of the dematching layer 3 and the piezoelectric element 4, in a case where the dematching layer 3 and the piezoelectric element 4 are diced using the cutting blade B, the first members 5 are also cut by the cutting blade B, but the first members 5 are formed of a material including abrasive grains. As a result, each time the dematching layer 3 and the piezoelectric element 4 are diced, the cutting blade B is subjected to a polishing treatment by the abrasive grains included in the first members 5. Therefore, it is possible to prevent the cut cross section of the piezoelectric element 4 from being damaged due to the abrasion of the cutting blade B.

As a result, it is possible to improve the reliability of the ultrasound probe.

The ultrasound probe according to Embodiment 1 of the present invention can be manufactured as follows.

First, the flexible circuit board 2 and the dematching layer 3 are sequentially bonded to the surface of the backing material 1 with an adhesive or the like, and the laminate L in which the signal electrode layer 4A, the piezoelectric body portion 4B, and the ground electrode layer 4C are sequentially laminated on the surface of the dematching layer 3 is bonded with an adhesive or the like.

Further, a pair of first members 5 are disposed and bonded on the flexible circuit board 2 to be adjacent to both end parts of the dematching layer 3 and the laminate L in the elevation direction D2.

Then, the metal foil 6 is bonded to the surfaces of the laminate L and the pair of first members 5, and the metal foil 6 bent at the corners of the pair of first members 5 is bonded to the side surfaces of the first members 5, the flexible circuit board 2, and the backing material 1. Thereafter, the acoustic matching layer 7 is bonded to the metal foil 6.

In this state, by relatively moving the cutting blade B in the elevation direction D2 while rotating the cutting blade B, the dematching layer 3, the laminate L, and the acoustic matching layer 7 are diced and divided into a plurality of portions in the azimuth direction D1, and the plurality of piezoelectric elements 4 are formed.

In this case, the pair of first members 5 are also divided into a plurality of pieces in the azimuth direction D1 in accordance with the plurality of piezoelectric elements 4, and the metal foil 6 of the portions disposed on the upper part of the piezoelectric element 4 and the side surfaces of the pair of first members 5 is also divided into a plurality of pieces in the azimuth direction D1 in accordance with the plurality of piezoelectric elements 4.

The pair of first members 5 are divided by the cutting blade B, and thus the abrasive grains included in the first members 5 polish the cutting blade B each time the dematching layer 3, the laminate L, and the acoustic matching layer 7 are diced.

Finally, the inside of the groove G formed by dicing is filled with the filler 8, and thus, the ultrasound probe shown in Fig. 1 is manufactured.

In Embodiment 1 described above, the metal foil 6 connected to the ground electrode layer 4C of the plurality of piezoelectric elements 4 extends to the side surface of the backing material 1 along the side surfaces of each of the first members 5 in the elevation direction D2, but the present invention is not limited thereto, and the metal foil 6 can also be configured to be bent along the front surface or the back surface of the flexible circuit board 2 and connected to a ground wiring layer (not shown) of the flexible circuit board 2. In this case, the metal foil 6 does not need to extend to the side surface of the backing material 1.

In addition, the acoustic matching layer 7 disposed on the metal foil 6 is also divided into a plurality of pieces in the azimuth direction D1 in accordance with the plurality of piezoelectric elements 4, but the present invention is not limited thereto. For example, the dicing may be performed before the acoustic matching layer 7 is disposed, whereby the dematching layer 3, the laminate L, the pair of first members 5, and the metal foil 6 may be divided into a plurality of portions in the azimuth direction D1, and then one acoustic matching layer 7, which extends to be longer in the azimuth direction D1, may be disposed on the plurality of divided piezoelectric elements 4 and the metal foil 6.

### Embodiment 2

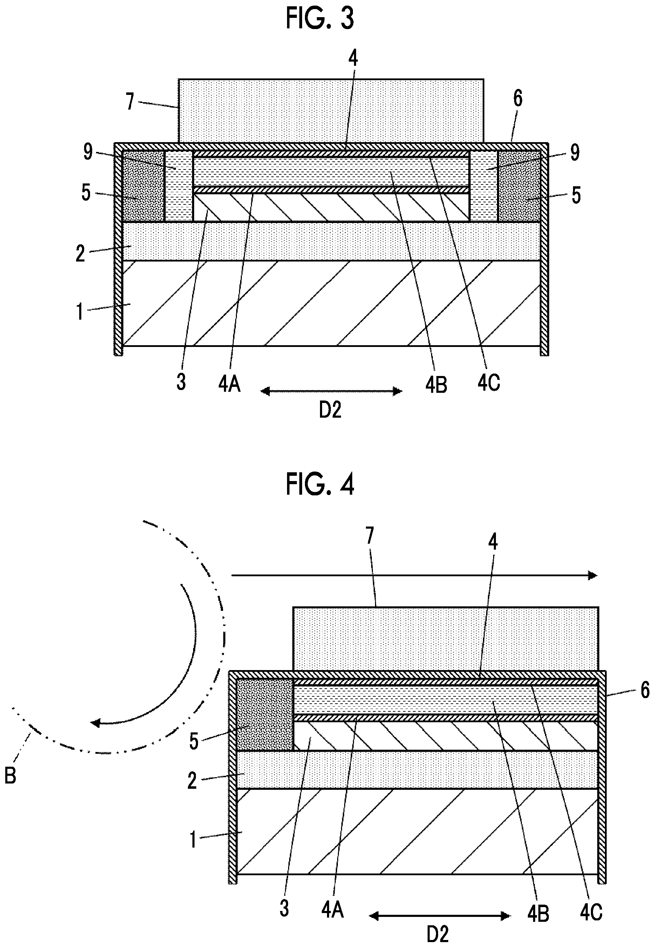

Fig. 3 shows a configuration of an ultrasound probe according to Embodiment 2 of the present invention. In the ultrasound probe of Embodiment 1, the second members 9 are each disposed between both end parts of the dematching layer 3 and the piezoelectric element 4 in the elevation direction D2 and the pair of first members 5, and the other members are the same as those of the ultrasound probe of Embodiment 1.

That is, the ultrasound probe according to Embodiment 2 includes the flexible circuit board 2 disposed on the backing material 1, the dematching layer 3 disposed on the flexible circuit board 2, and the plurality of piezoelectric elements 4 disposed on the dematching layer 3, and the pair of first members 5 are disposed at both end parts of the dematching layer 3 and the piezoelectric element 4 in the elevation direction D2 via a pair of second members 9 formed on the flexible circuit board 2.
the metal foil 6 is disposed on the plurality of piezoelectric elements 4, the pair of second members 9, and the pair of first members 5, and the acoustic matching layer 7 is disposed on the metal foil 6.

Each of the pair of second members 9 has the same height as the first members 5 and has an acoustic attenuation rate higher than an acoustic attenuation rate of the first members 5. Specifically, the second members 9 can be formed of a buffer material including a silicone resin or an epoxy resin, similarly to the filler 8 that fills the groove G.

In this way, by interposing the second member 9 having the acoustic attenuation rate higher than the acoustic attenuation rate of the first members 5 between the piezoelectric element 4 and the first members 5, it is possible to suppress the influence of the first members 5 on the acoustic characteristics of the piezoelectric element 4 due to the direct contact, and it is possible to further improve the reliability of the ultrasound probe.

Even in the ultrasound probe according to Embodiment 2, even in a case where the flexible circuit board 2 is formed longer than the dematching layer 3 and the piezoelectric element 4 in the elevation direction D2, a gap is not generated at both end parts of the dematching layer 3 and the piezoelectric element 4 in the elevation direction D2, and it is possible to prevent the piezoelectric element 4 from being damaged due to fluttering of the cutting blade B in a case of dividing the piezoelectric element 4 into the plurality of piezoelectric elements 4 by dicing using the cutting blade B. In addition, it is not necessary to increase the lengths of the dematching layer 3 and the piezoelectric element 4 in the elevation direction D2 in accordance with the length of the flexible circuit board 2, and it is not necessary to change the acoustic design.

Further, each time the dematching layer 3 and the piezoelectric element 4 are diced, the abrasive grains included in the first members 5 perform a polishing treatment on the cutting blade B, and it is possible to prevent the cut cross section of the piezoelectric element 4 from being damaged due to the abrasion of the cutting blade B.

### Embodiment 3

Fig. 4 shows a configuration of an ultrasound probe according to Embodiment 3 of the present invention. In the ultrasound probe of Embodiment 1, instead of disposing the pair of first members 5 at both end parts of the dematching layer 3 and the piezoelectric element 4 in the elevation direction D2, the first member 5 formed on the flexible circuit board 2 is disposed only at one end part of the dematching layer 3 and the piezoelectric element 4 in the elevation direction D2. Other members are the same as those of the ultrasound probe of Embodiment 1.

That is, the ultrasound probe according to Embodiment 3 includes the flexible circuit board 2 disposed on the backing material 1, the dematching layer 3 disposed on the flexible circuit board 2, and the plurality of piezoelectric elements 4 disposed on the dematching layer 3, and the first member 5 is disposed at one end part of the dematching layer 3 and the piezoelectric element 4 in the elevation direction D2. Further, the metal foil 6 is disposed on the plurality of piezoelectric elements 4 and the first members 5, and the acoustic matching layer 7 is disposed on the metal foil 6.

Even in the ultrasound probe according to Embodiment 3, even in a case where the flexible circuit board 2 is formed longer than the dematching layer 3 and the piezoelectric element 4 in the elevation direction D2, a gap is not generated at the end parts of the dematching layer 3 and the piezoelectric element 4 in the elevation direction D2, and it is possible to prevent the piezoelectric element 4 from being damaged due to fluttering of the cutting blade B in a case of dividing the piezoelectric element 4 into the plurality of piezoelectric elements 4 by dicing using the cutting blade B. In addition, it is not necessary to increase the lengths of the dematching layer 3 and the piezoelectric element 4 in the elevation direction D2 in accordance with the length of the flexible circuit board 2, and it is not necessary to change the acoustic design.

Further, each time the dematching layer 3 and the piezoelectric element 4 are diced, the abrasive grains included in the first members 5 perform a polishing treatment on the cutting blade B, and it is possible to prevent the cut cross section of the piezoelectric element 4 from being damaged due to the abrasion of the cutting blade B.

In order to prevent the piezoelectric element 4 from being damaged due to fluttering of the cutting blade B during dicing, as shown in Fig. 4, it is desirable to relatively move the cutting blade B in the elevation direction D2 from one end part where the first member 5 is disposed toward the other end part where the first member 5 is not disposed, among both end parts of the dematching layer 3 and the piezoelectric element 4 in the elevation direction D2.

In addition, Embodiment 3 can be applied to the ultrasound probe of Embodiment 2, and the second members 9 and the first members 5 can be disposed only at one end part of the dematching layer 3 and the piezoelectric element 4 in the elevation direction D2.

### Embodiment 4

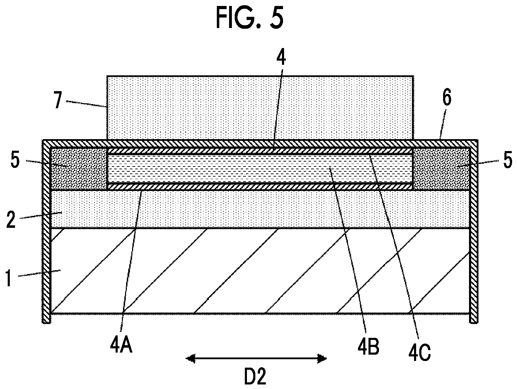

Fig. 5 shows a configuration of an ultrasound probe according to Embodiment 4 of the present invention. In the ultrasound probe of Embodiment 1, the dematching layer 3 is omitted, and the plurality of piezoelectric elements 4 are directly disposed on the surface of the flexible circuit board 2. Other members are the same as those of the ultrasound probe of Embodiment 1.

That is, the ultrasound probe according to Embodiment 4 includes the flexible circuit board 2 disposed on the backing material 1, the plurality of piezoelectric elements 4 disposed on the flexible circuit board 2, and the pair of first members 5 disposed at both end parts of the piezoelectric element 4 in the elevation direction D2. Further, the metal foil 6 is disposed on the plurality of piezoelectric elements 4 and the pair of first members 5, and the acoustic matching layer 7 is disposed on the metal foil 6.

Even in the ultrasound probe according to Embodiment 4, even in a case where the flexible circuit board 2 is formed to be longer than the piezoelectric element 4 in the elevation direction D2, a gap is not generated at the end parts of the piezoelectric element 4 in the elevation direction D2, and it is possible to prevent the piezoelectric element 4 from being damaged due to fluttering of the cutting blade B in a case of dividing the piezoelectric element 4 into the plurality of piezoelectric elements 4 by dicing using the cutting blade B. In addition, it is not necessary to increase the length of the piezoelectric element 4 in the elevation direction D2 in accordance with the length of the flexible circuit board 2, and it is not necessary to change the acoustic design.

Further, each time the piezoelectric element 4 is diced, the abrasive grains included in the first members 5 perform a polishing treatment on the cutting blade B, and it is possible to prevent the cut cross section of the piezoelectric element 4 from being damaged due to the abrasion of the cutting blade B.

However, as in Embodiment 1, in a case where the plurality of piezoelectric elements 4 are disposed on the surface of the dematching layer 3, the dematching layer 3 can reflect the ultrasonic waves emitted from the plurality of piezoelectric elements 4 rearward to the front side, and a high-performance ultrasound probe can be configured.

In addition, the dematching layer 3 can be omitted by applying Embodiment 4 to the ultrasound probe of Embodiment 2 or Embodiment 3.

### Explanation of References

1: backing material
2: flexible circuit board
3: dematching layer
4: piezoelectric element
4A: signal electrode layer
4B: piezoelectric body portion
4C: ground electrode layer
5: first member
6: metal foil
7: acoustic matching layer
8: filler
9: second member
D1: azimuth direction
D2: elevation direction
G: groove
L: laminate
B: cutting blade

## Claims

1. An ultrasound probe in which a plurality of piezoelectric elements are arranged in an array on a backing material along an azimuth direction, the ultrasound probe comprising:
a flexible circuit board disposed between the backing material and the plurality of piezoelectric elements,
wherein each of the plurality of piezoelectric elements consisting of a laminate in which a signal electrode layer, a piezoelectric body portion, and a ground electrode layer are sequentially laminated in a lamination direction on a surface of the flexible circuit board,
the signal electrode layer is electrically connected to the flexible circuit board,
the flexible circuit board is longer than the piezoelectric body portion in an elevation direction,
first members consisting of an adhesive in which abrasive grains are dispersed are disposed at end parts of the laminate in the elevation direction, and
the ground electrode layer is electrically connected to the flexible circuit board via metal foil that extends from a surface of the laminate to surfaces of the first members and side surfaces of the first members in the elevation direction.

2. The ultrasound probe according to claim 1,
wherein the first members are each disposed at both end parts of the laminate in the elevation direction.

3. The ultrasound probe according to claim 1 or 2,
wherein the first members have an acoustic attenuation rate equal to or higher than an acoustic attenuation rate of the backing material.

4. The ultrasound probe according to any one of claims 1 to 3,
wherein the first members consist of a vitreous adhesive or a ceramic adhesive in which any of a white alumina abrasive, a green silicon carbide, or a resinoid is dispersed as the abrasive grains.

5. The ultrasound probe according to any one of claims 1 to 4,
wherein second members having an acoustic attenuation rate higher than an acoustic attenuation rate of the first members are disposed between the end parts of the laminate in the elevation direction and the first members.

6. The ultrasound probe according to claim 5,
wherein the second members consist of a buffer material.

7. The ultrasound probe according to claim 6,
wherein the buffer material consists of a silicone resin or an epoxy resin.

8. The ultrasound probe according to any one of claims 1 to 7, further comprising:
a dematching layer disposed between the flexible circuit board and the plurality of piezoelectric elements and having an acoustic impedance higher than an acoustic impedance of the piezoelectric body portion.
